# EUROPEAN PATENT APPLICATION

(11) **EP 4 265 594 A1**
(43) Date of publication of application: **25.10.2023**
(21) Application number: 20966268.3
(22) Date of filing: 21.12.2020
(51) Int. Cl.: C07C 237/20, C07C 237/06, C07C 237/02, A61K 31/165, A61P 23/00

(54) **DRUG CONJUGATE, PREPARATION METHOD THEREFOR, AND USE THEREOF**

(71) Applicant: Lipont Pharmaceuticals Inc., Richmond, BC V6V 3B7 (CA); Wang, Jiucheng, Xi'an, Shaanxi 710075 (CN)
(72) Inventor: CAO, Suoding, Richmond, British Columbia V6V 3B7 (CA); WANG, Jiucheng, Shaanxi 710075 (CN); YANG, Ross, Richmond, British Columbia V6V 3B7 (CA); LIU, Cecily, Richmond, British Columbia V6V 3B7 (CA); LU, Wudang, Shaanxi 710077 (CN); JIAO, Yaqi, Shaanxi 710077 (CN); CHO, Tara, Richmond, British Columbia V6V 3B7 (CA); YANG, Fengshou, Richmond, British Columbia V6V 3B7 (CA)
(74) Representative: Sweetinburgh, Mark Roger
(86) International application number: PCT/CN2020/138133
(87) International publication number: WO 2022/133687

(57) **Abstract**

A drug conjugate or a pharmaceutically acceptable salt thereof. Gabapentin or a derivative thereof is coupled with propofol or a derivative thereof. Compared with a parent drug, the water solubility and bioavailability of the drug conjugate are effectively improved, and the toxic and side effects of the drug are reduced. Such a drug conjugate has a pharmaceutical effect and can be used for treating and/or preventing central nervous diseases, such as convulsion/epilepsy, migraine, pain and depression.

## Description

### TECHINICAL FIELD

The present invention relates to a class of drug-drug conjugates (Drug-Drug Conjugate), more particularly to the preparation of drug conjugates formed by propofol (or derivatives thereof) and gabapentin (or derivatives thereof) and salts thereof and crystallization methods. The present invention also relates to the use of the above drug conjugates and their salts in the treatment or prevention of central nervous system diseases, such as convulsions/epilepsy, migraine and depression.

### BACKGROUND OF THE INVENTION

Drug conjugates are a proven approach to emerging drug innovations. Drug conjugates are new molecular entities formed by covalently linking two or more biologically active single drugs. The main purpose of this design is to circumvent the limitations of most single drugs in clinical use. Compared with traditional drugs, drug conjugates have some special advantages, including improved solubility, improved bioavailability, prolonged half-life and prolonged treatment period, reduced toxicity, and reduced drug resistance.

Propofol and gabapentin are both FDA-approved active compounds with great utility in the treatment or prevention of central nervous system disorders. Propofol is an intravenous short-acting anesthetic that is widely used in the induction and maintenance of anesthesia. Compared with other anesthetics, propofol has the advantages of rapid onset of action, high clearance rate, rapid awakening, and complete recovery of neurophysiological functions. In addition, the various pharmacological effects of propofol have been shown to be useful in the treatment of a variety of clinical conditions, including migraine, nausea, pain, and anxiety. However, due to the limited water solubility, poor pharmacokinetic characteristics and hepatic first-pass effect of propofol, the oral bioavailability of propofol is very low, and it is difficult to achieve the lowest effective dose of the drug, which hinders its wider clinical application, especially in the central nervous system.Gabapentin is a compound that is structurally similar to the neurotransmitter gamma-aminobutyric acid (GABA). It is primarily used to treat neuropathic pain and as an adjunctive treatment for partial-onset seizures in adults with epilepsy. Clinical studies have shown a potential role for gabapentin in the treatment of restless legs syndrome, anxiety, and other neurological disorders. However, due to the high polarity and poor lipid solubility of gabapentin, it is difficult to penetrate the blood-brain barrier, and it is prone to absorb dose saturation during clinical use, resulting in dose-dependent kinetics, which greatly limits the application of gabapentin in other central nervous system diseases.

So far, in the process of exploring new applications of propofol and gabapentin in central nervous system diseases, there have been many studies on improving the pharmacokinetics of these two compounds, most of which are achieved by improving the pharmacokinetics of these two compounds, design or formulation innovations in prodrugs (eg, non-emulsion formulations of propofolum with cyclodextrins and micelles, amino acid ester and phosphate ester derivatives of propofol) , Schiff bases of gabapentin), however, Their success has been very limited. Because propofol and gabapentin each has complex pharmacological mechanisms of action and very different pharmacokinetic profiles, it is difficult to administer the two active substances in a single pharmaceutical formulation. When people tried to combine propofol and gabapentin in two different formulations, the ideal synergistic effect of the two drugs was not observed to improve the efficacy.

In order to overcome the respective limitations of propofol and gabapentin in the pharmacokinetics of the two active substances, and to discover and improve their new applications in the prevention and treatment of central nervous system diseases, the application of drug-drug conjugates provides another whole new way. The new molecular entities generated by coupling propofol and gabapentin in a stable covalent bond form can effectively improve the hydrophilic and lipophilic properties of the original two synthon active groups, thereby improving the pharmacokinetic properties of the two and even more easy to formulate medicines and reduce toxic and side effects. As a new molecular entity, the newly obtained conjugate exerts pharmacological effects in vivo, and may have a completely different pharmacological mechanism of action from propofol and gabapentin, and thus may develop its new application in central nervous system diseases.

### SUMMARY OF THE INVENTION

The purpose of the present invention is to provide a class of propofol-gabapentin drug conjugates and preparation methods and uses thereof. The first aspect of the present invention provides a drug conjugate or a pharmaceutically acceptable salt thereof, the structure of the drug conjugate is shown in formula (I):

Dₚ-L-D_{g} (I)

wherein, D_{g} is gabapentin or its derivative; L is selected from carbonyl group or may not exist; Dₚ is selected from propofol or its derivative;
preferably, the structure of the pharmaceutically acceptable salt of the drug conjugate is shown in formula (II):

   Dₚ-L-D_{g}•A (II)
wherein, A is a pharmaceutically acceptable acid anion, preferably is selected from one or more of the following: sulfate, phosphate, hydrochloride, hydrobromide, acetate, oxalate, citrate, succinic acid Salt, gluconate, tartrate, p-toluenesulfonate, benzenesulfonate, mesylate.

The drug conjugate or a pharmaceutically acceptable salt thereof according to the first aspect of the present invention, wherein, D_{g} is gabapentin or its derivative thereof as shown in formula (III): 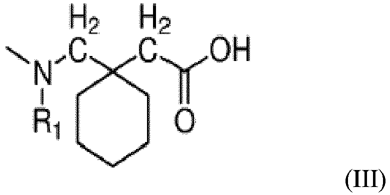 wherein, R₁ is a methyl group or a hydrogen atom.

The drug conjugate or a pharmaceutically acceptable salt thereof according to the first aspect of the present invention, wherein, D_{g} is gabapentin or its derivative thereof as shown in formula (IV): 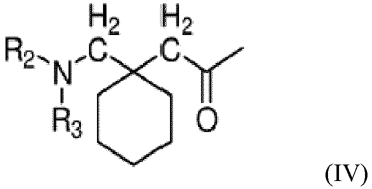 wherein, R₂ and R₃ are independently selected from methyl group or hydrogen atoms.

The drug conjugate or a pharmaceutically acceptable salt thereof according to the first aspect of the present invention, wherein, D_{g} is a derivative of gabapentin represented by formula (V): 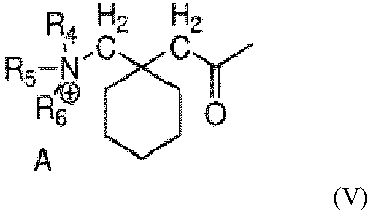 wherein, R₄, R₅ and R₆ are independently selected from methyl group or hydrogen atoms.

The drug conjugate or a pharmaceutically acceptable salt thereof according to the first aspect of the present invention, wherein, Dₚ is propofol or its derivative thereof as shown in formula (VI): 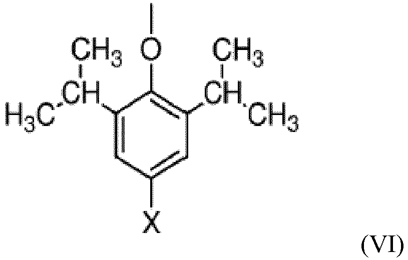 wherein, X is a halogen atom or a hydrogen atom.

The second aspect of the present invention provides the preparation method of the drug conjugate or a pharmaceutically acceptable salt thereof according to the first aspect, the method comprises the following steps:
the drug conjugate is prepared by suspending Dp and Dg in an organic solvent;
preferably, the organic solvent is selected from one or more of the following: dichloromethane, tetrahydrofuran/water, diethyl ether, methanol, ethanol, isopropanol, benzene, toluene; preferably dichloromethane and/or tetrahydrofuran/water.

The method according to the second aspect of the present invention, wherein, the method comprises the following steps: preparing the derivative D_{g} of gabapentin as described in formula (VII): 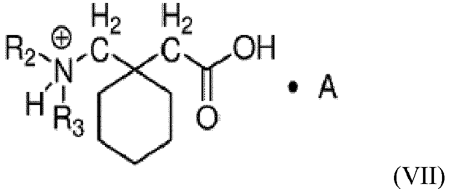
preferably, the step comprises: dissolving gabapentin in an aldehyde solution, and adding a reducing agent to prepare a gabapentin derivative;
more preferably, the aldehyde solution is an aqueous formaldehyde solution, and the amount of the aldehyde is 1-5 molar equivalents, preferably 3 molar equivalents; and/or the reducing agent is formic acid, and the amount of the reducing agent is 1-5 molar equivalents, preferably is 4 molar equivalents;
further preferably, the reaction temperature is 60-90 °C; and/or the reaction time is 3-12 hours.

The method according to the second aspect of the present invention, wherein, the method comprises the following steps: preparing the derivative Dₚ of propofol represented by formula (VIII): 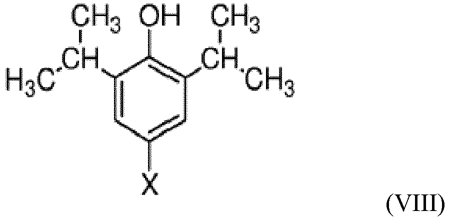
preferably, the step comprises: dissolving propofol in an organic solvent, adding a nucleophile, a base and an oxidizing agent to prepare the propofol derivative;
more preferably, the organic solvent is methanol, the nucleophile is sodium iodide, the base is sodium hydroxide, and/or the oxidant is sodium hypochlorite;
further preferably, the reaction temperature is 0°C and/or the reaction time is 1-3 hours;
more further preferably, the reaction is quenched with sodium thiosulfate after completion.

The method according to the second aspect of the present invention, wherein, when the D_{g} is coupled with the N-terminus of gabapentin, first prepare Boc-gabapentin and then react with Dp;
preferably, the preparation method of the Boc-gabapentin comprises the following steps: stirring and suspending the gabapentin in an organic solvent, adding NaHCOs and BoczO in turn to react to obtain the Boc-gabapentin;
more preferably, the organic solvent is preferably 1:1 tetrahydrofuran/water; the NaHCOs is used in an amount of 1 to 5 molar equivalents, preferably 3 molar equivalents; and/or the Boc₂O is used in an amount of 1 to 2 molar equivalents, preferably 1.1 molar equivalents.

The method according to the second aspect of the present invention, wherein, the method further comprises the step of extracting the crude product with an organic solvent;
preferably, the organic solvent is selected from one or more of the following:
dichloromethane, chloroform, diethyl ether, methanol, ethanol, isopropanol, benzene, toluene; preferably dichloromethane.

The third aspect of the present invention provides a pharmaceutical composition, the pharmaceutical composition comprises the drug conjugate or a pharmaceutically acceptable salt thereof according to the first aspect or the drug conjugate or a pharmaceutically acceptable salt thereof prepared by the preparation method according to the second aspect;
preferably, the dosage form of the pharmaceutical composition is selected from one or more of the following: tablets, capsules, injections, liposomes, and polymer microspheres.

The fourth aspect of the present invention provides use of the drug conjugate or a pharmaceutically acceptable salt thereof according to the first aspect or the drug conjugate or a pharmaceutically acceptable salt thereof prepared by the preparation method according to the second aspect in the preparation of medicaments for the treatment and/or prevention of central nervous system diseases;
preferably, the central nervous system disease is selected from one or more of the following: convulsions, epilepsy, migraine, pain, depression.

The fifth aspect of the present invention provides a method for treating a central nervous system disease, the method comprises: administering the drug conjugate or a pharmaceutically acceptable compound thereof according to the first aspect or a pharmaceutical composition according to the third aspect to a subject in need;
preferably, the central nervous system disease is selected from one or more of the following: convulsions, epilepsy, migraine, pain, depression.

The sixth aspect of the present invention provides a medicament for the treatment and/or prevention of central nervous system diseases, the medicament comprises the drug conjugate or a pharmaceutically acceptable salt thereof according to the first aspect or the drug conjugate or a pharmaceutically acceptable salt thereof prepared by the preparation method according to a the third aspect.

In an attempt to discover novel and effective new drug entities based on propofol, the inventors formed drug-drug conjugates by conjugating gabapentin or a derivative thereof with propofol. So far, the use of drug-drug conjugates is mostly limited to anticancer drugs, and there is no relevant report in the drug research of the central nervous system. In the present invention, the inventors coupled a gabapentin or a derivative thereof with a propofol or a derivative thereof via a linker (or in some cases no linker) to form a drug-drug conjugate and its crystalline form.

The inventors found in the water solubility experiments of the synthesized drug conjugates that the water solubility of example compounds 1, 2 and 5 were all greater than 10 mg/ml, showing good water solubility.

The inventors found in the liposolubility experiments of the synthesized drug conjugates that all the example compounds 1-6 had good liposolubility characteristics, and their fat-water partition coefficient logP was 1.10, 1.66, 2.38, 3.52, 1.37, 1.54. Generally, whether the drug development of the central nervous system is successful or not, in addition to the compounds need to have good biological activity and metabolic properties and low toxicity, they also need to have good liposolubility to overcome the blood-brain barrier (blood-brain barrier, BBB), to achieve sufficient exposure in the central system. The drug conjugates in the present invention all have good liposolubility characteristics, and such good liposolubility characteristics can effectively improve the penetration rate of such drugs through the blood-brain barrier, thereby improving the bioavailability of the drugs.

In the in vitro plasma decomposition experiment, the inventors found that a conjugate of the present invention has good stability under neutral and acidic plasma conditions, which indicates that this conjugate is more likely to act as a new molecular entity exerts pharmacodynamic effects in vivo, and this type of conjugate as a new molecular entity may have more beneficial pharmacological properties than we expected. For example, improving the plasma stability of the compound can prolong the action time of the drug in vivo, increase the exposure in vivo, reduce the clearance rate of the compound, improve the bioavailability, and then improve its pharmacokinetic and pharmacodynamic properties.

It is found in the present invention that such drug conjugates have medicinal effects and can be used to treat and/or prevent central nervous system diseases, such as convulsions/epilepsy, migraine, pain and depression, etc.

The present invention relates to drug conjugates in the form of drug-drug conjugate products, which in particular exhibit activity in the treatment and/or prevention of convulsions/epileptics, migraine, depression.

The present invention also relates to a method for synthesizing such a molecule, which has few synthesis steps and is therefore easy to implement on an industrial scale.

The present invention also relates to the therapeutic use of such molecules, in particular their use in the treatment and/or prevention of convulsions/epilepsy, migraine, depression.

The drug conjugates of the present invention are characterized in that they consist of coupling products according to formulae (I) and (II):

Dₚ-L-D_{g} (I)

Dₚ-L-D_{g}•A (II)

wherein.
D_{g} is gabapentin or a derivative thereof;
Dₚ is propofol and/or its derivatives;
L is selected from carbonyl group, or may not exist;

A represents a pharmaceutically acceptable acid anion such as: sulfate, phosphate, hydrochloride, hydrobromide, acetate, oxalate, citrate, succinate, gluconate, tartrate, p-toluenesulfonate, benzenesulfonate, methanesulfonate, etc.

In the preferred compound of the present invention, D_{g} represents gabapentin or its N-terminal partial derivatized product, which is linked as shown in the following formula III: 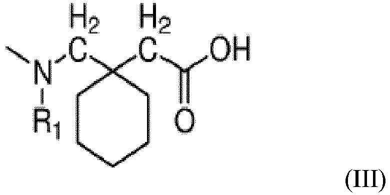 wherein, R₁ is a methyl group or a hydrogen atom.

In the preferred compound of the present invention, D_{g} represents gabapentin or its derivative represented by the following formula IV: 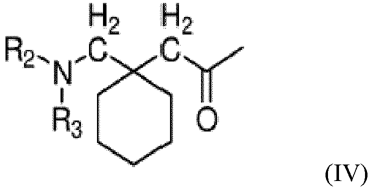 wherein, R₂ and R₃ may be the same or different and are methyl group or hydrogen atoms.

In the present invention, preferably, D_{g} is gabapentin or its derivative represented by the following formula V: 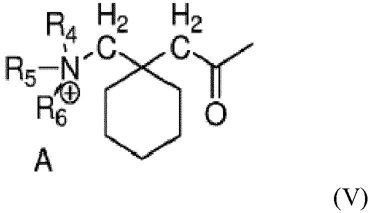 wherein, R₄, R₅ and R₆ which may be the same or different, are methyl group or hydrogen atoms, and A is as defined above.

In the present invention, preferably, Dₚ is propofol or its derivative of the following formula VI: 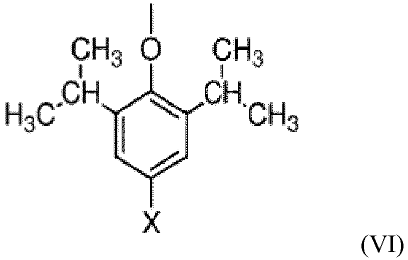 wherein, X is a halogen atom or a hydrogen atom.

The present invention also relates to the preparation method of related gabapentin and propofol derivatives.

### a: Preparation of gabapentin derivatives represented by formula VII

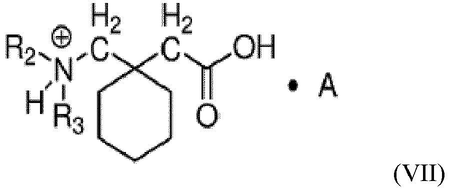

wherein, A, R₂ and R₃ are as described above.

According to an embodiment of the present invention, gabapentin derivatives are prepared by dissolving gabapentin in an aldehyde solution (preferably aqueous formaldehyde) and then adding a reducing agent (preferably formic acid). The reaction is preferably stirred at a temperature of 60°C to 90°C for 3 to 12 hours. The amount of aldehyde is preferably 3 molar equivalents, and the amount of reducing agent is preferably 4 molar equivalents. After cooling, the mixture is condensed under reduced pressure and then admixed with an acid(preferably hydrochloric acid). The solution was stirred for 1-12 hours. The product obtained by recrystallization.

### b: Preparation of propofol derivatives represented by formula VIII

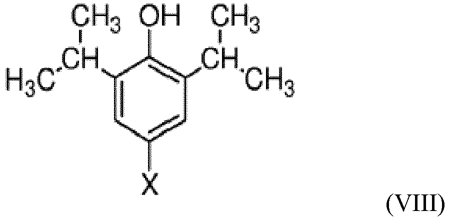

wherein, X is as described above.

According to embodiments of the present invention, propofol derivatives are prepared by dissolving propofol in an organic solvent (preferably methanol). A nucleophile (preferably sodium iodide), and a base (preferably sodium hydroxide) are added. The amount of nucleophile is preferably 1 molar equivalent, and the reaction is preferably carried out under stirring at 0°C. An oxidizing agent (preferably sodium hypochlorite) is added slowly at 0°C. The reaction was run for 1-3 hours and then quenched with sodium thiosulfate. The pH of the solution was adjusted to neutrality with hydrochloric acid. The crude product is obtained by extraction with an organic solvent such as diethyl ether; further purification by silica gel chromatography yields the product.

### c: The coupling of the gabapentin derivative of formula VII with propofol or the derivative of formula VIII is the following conjugate of formula IX

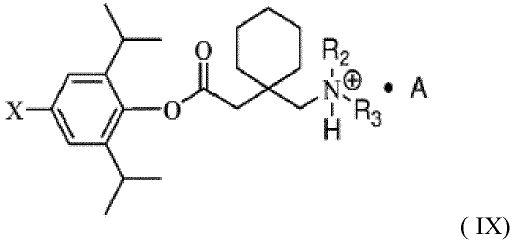

wherein, X, A, R₂, R₃ are as described above.

According to the embodiment of the present invention, the coupling reaction is carried out by dissolving the gabapentin derivative represented by formula VII and the propofol or propofol derivative represented by formula VIII in an organic solvent (preferably dichloromethane). The amount of the acylating agent is preferably 2 molar equivalents. The mixture is stirred for reaction at low temperature, preferably in a cooling bath at 0°C for 10 to 30 minutes. An acylating agent (preferably oxalyl chloride) is added together with an amide catalyst (preferably dimethylformaldehyde). The solution is stirred at 0 to 20°C for 5 to 16 hours to obtain the acid chloride gabapentin derivative.

The obtained crude product is redissolved in an organic solvent (preferably dichloromethane) in an ice bath, then an organic base (preferably pyridine) is added, and then propofol or a propofol derivative is slowly added. The amount of the organic base is preferably 2 to 3 equivalents. The mixture is stirred at 0-20°C for 5-12 hours and then washed with aqueous hydrochloric acid, preferably pH 1 aqueous hydrochloric acid. The crude product was obtained by extraction with dichloromethane, and the crude product was purified by silica gel chromatography and finally recrystallized to the final product.

### d: Gabapentin is coupled with propofol or its derivative represented by formula VIII to prepare a conjugate represented by formula X

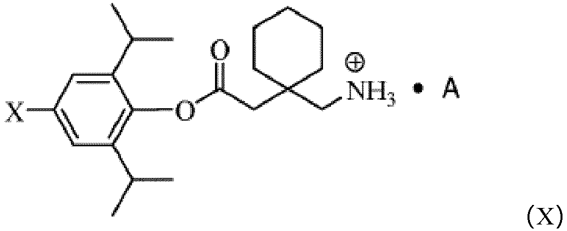

wherein, X and A1 are as described above.

According to the embodiment of the present invention, the conjugate is prepared by suspending gabapentin and propofol or the propofol derivative represented by formula VIII in an organic solvent, and the organic solvent is preferably 1:1 tetrahydrofuran/water. The gabapentin suspension is stirred in an ice bath, followed by the addition of a base (preferably sodium bicarbonate), followed by an amine protecting agent (preferably di-tert-butyl dicarbonate). The amount of amine protecting is preferably 1 molar equivalent. The reaction solution was heated to 20°C and stirred overnight. Extraction with organic solvent (preferably diethyl ether) then acidifies the aqueous layer to pH 7 by careful addition of potassium bisulfate at 0°C. The resulting aqueous mixture was extracted with dichloromethane. The two organic phases were combined and dried over sodium sulfate, and the solvent was removed under reduced pressure. The white solid can be used for the next coupling reaction without further purification.

Dissolve the white solid obtained above and propofol or its derivative in an organic solvent (preferably dichloromethane), add an amine catalyst (preferably 4-dimethylaminopyridine), and at 0°C, slowly add the coupling agent (preferably N, N '-dicyclohexylcarbodiimide) dissolved in the same solvent to the mixture and stir for 5-18 hours at 20 °C. The reaction solution was filtered, and the solvent was removed under reduced pressure. The resulting crude coupling product was purified by silica gel chromatography.

The product obtained above is dissolved in an acidic solution, preferably diethyl ether in hydrogen chloride. The solution was stirred for 1-5 days, then the solvent was removed and the final product was obtained by recrystallization.

### e: Coupling of gabapentin with propofol or derivatives represented by formula VIII to prepare XI

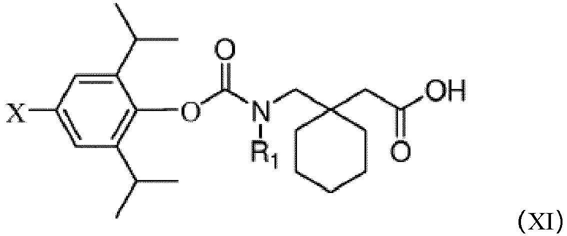

wherein, X and R1 are as described above.
(1)According to the embodiment of the present invention, by suspending gabapentin in an organic solvent (preferably dichloromethane), a conjugate of gabapentin and propofol or a propofol derivative represented by formula (VIII) is prepared. The gabapentin suspension is cooled to 0-15°C, the amine (preferably diisopropylethylamine) is added, and then the amine protecting agent (preferably chlorotrimethylsilane) is added. The solution was then stirred for 1-2 hours before proceeding to step (3).
(2)In another reaction vessel, dissolve propofol or its derivatives in the same solvent as the last step at 0°C. Trichloromethyl carbonate (triphosgene) is added, preferably in an amount of 0.4 molar equivalents; then add an amine, preferably diisopropylethylamine. The reaction was stirred at 20°Cfor 12-16 hours before proceeding to step (3).
(3)The reaction solution of step (2) was evaporated under reduced pressure at 10-25 °C to near dryness, then redissolved in the same organic solvent as above, the reaction solution of step (1) was gradually added dropwise, and then stirred at 25°C for 12 hours. The reaction solution was extracted with dichloromethane to obtain a crude product, which was further purified by column chromatography.

### f: Preparation of the quaternary ammonium salt of the conjugate of formula XII from step e

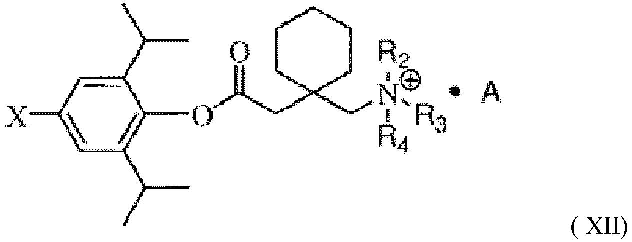

wherein X, A, R2, R3 and R4 are as described above.

According to an embodiment of the present invention, the free amine is extracted from the aqueous layer by dissolving the conjugate obtained from step C in water, adding sodium carbonate until the pH of the solution reaches 8, and extracting with an organic solvent (preferably dichloromethane). After removing the solvent under reduced pressure, methyl iodide was added as a solvent and reagent, stirred for 12 hours, and recrystallized to obtain the final product.

Migraine as described in the present invention refers to the following conditions: throbbing headache on one or both sides of the head, each attack lasting between hours and days. The above conditions usually cause nausea, vomiting, and photophobia.

In the examples of the present invention, the nitroglycerin-induced migraine mouse model established by subcutaneous injection of nitroglycerin is selected, which is the most classic migraine model at present, and has been widely used in the experimental and evaluation of drugs for treating migraine both domestically and internationally.

Behaviorally, scratching the head five times or scratching the face with the forelimb more than five times is considered to be a migraine-related behavior induced by nitroglycerin; in the hot plate test, raising the hindlimb and licking the hindfoot can be used as indicators to observe the analgesic effect.

The present invention evaluates the effect of the drug conjugate on migraine induced by nitroglycerin in mice, and takes the pain threshold of hot plate test and head scratching behavior as observation indicators. The results show that the drug conjugate formed by gabapentin or its derivatives and propofol or its derivatives, or a pharmaceutically acceptable salt thereof, can effectively relieve the pain caused by nitroglycerin-induced migraine.

Longitudinal studies have shown that depression is usually a progressive lifelong disorder that manifests in recurrent, or prolonged episodes (chronic depression). In the example of the present invention, the depression mouse model established by subcutaneous injection of lipopolysaccharide is selected, which is widely used in the experiment and evaluation of drugs for treating depression.

The open field test instrument and related computer software were used to count the total migration distance of each mouse within 5 minutes in the open field (50 × 50 × 50 cm) to evaluate the effect of the test drug on the spontaneous activity of the mice; the forced swimming test (FST) was utilized, in which mice were placed in a transparent cylinder filled with warm water (about 25 °C) for 6 minutes. The mice experienced behaviors such as struggling, forced swimming, and floating, and the time the mice stopped struggling and floating on the water surface was considered as a state of despair caused by depression.

The test results in the examples of the present invention show that the drug conjugates formed by gabapentin or its derivatives and propofol or its derivatives, or pharmaceutically acceptable salts thereof, can effectively alleviate depression in mouse models induced by lipopolysaccharide.

The present invention evaluates the effect of this drug conjugate on convulsions/epileptic mice induced by electrical stimulation. The results showed that this drug conjugate significantly improved the anti-electroconvulsant ability of mice at an effective dose, and was effective for convulsions/epilepsy.

The results of Test Example 8-10 of the present invention show that the drug conjugates formed by gabapentin or its derivatives and propofol or its derivatives, or pharmaceutically acceptable salts thereof, has better sedative and antidepressant effects compared to using propofol alone or gabapentin+propofol in combination.

The drug conjugates and their pharmaceutically acceptable salts prepared in the present invention can be made into any dosage form. Preferably, it can be prepared in the form of tablets, capsules, injections, lyophilized powders, emulsions, liposomes or polymer microspheres.

### BRIEF DESCRIPTION OF THE DRAWINGS

Hereinafter, the embodiments of the present invention will be described in detail with reference to the drawings, in which:
Figure 1 shows the results of comparing the weight gain of animals among the groups in Test Example 1.
Figure 2 shows the comparison results of hind paw licking time among groups measured by the hot plate method in Test Example 1.
Figure 3 shows the comparison of the total head scratching times among the groups in Test Example 1.
Figure 4 shows the results of one-way ANOVA comparison of animal body weight gain among each group in Test Example 2.
Figure 5 shows the comparison results of one-way ANOVA of the total distance of open field migration among each group in Test Example 2.
Figure 6 shows the comparison results of one-way ANOVA of FST immobility time among each group in Test Example 2.
Figure 7 shows the results of the one-way ANOVA comparison of the weight gain values of the mice in each group in Test Example 3.
Figure 8 shows the comparison results of one-way ANOVA of the threshold voltage increase values measured before and after administration of each group of mice in Test Example 3.
Figure 9 shows the comparison results of one-way ANOVA of changes in the preference of sugar water in each group of mice before and after administration in Test Example 9.
Figure 10 shows the comparison results of one-way ANOVA of changes in the immobility time (s) of forced swimming in each group of mice in Test Example 10 before and after administration.

### BEST MODE FOR CARRYING OUT THE INVENTION

The present invention is further described below through specific examples, however, it should be understood that these examples are only used for more detailed description, and should not be construed to limit the present invention in any form.

This section provides a general description of the materials and test methods used in the tests of the present invention. While many of the materials and methods of operation used for the purposes of the present invention are known in the art, the present invention is described in as much detail as possible. It is clear to those skilled in the art that, in the context, if not specifically stated, the materials and methods of operation used in the present invention are well known in the art.

The reagents and instruments used in the following examples are as follows:
Reagents: gabapentin was purchased from AK Scientific (CA, USA); THF, NaHCO₃, Boc₂O, diethyl ether, KHSO₄, dichloromethane, Na₂SO₄, propofol, 4-dimethylaminopyridine, N,N'-dicyclohexylcarbon diimine, ethyl acetate, hexane, diethyl ether, methanol, sodium iodide, sodium hydroxide, sodium hypochlorite, sSodium thiosulfate, hydrochloric acid, diisopropylethylamine, chlorotrimethylsilane, trichloromethane carbonate base ester, formic acid, formaldehyde, acetone, oxalyl chloride, dimethylformaldehyde, pyridine, aqueous CMC-Na, flunarizine hydrochloride, nitroglycerin, purchased from Sigma-Aldrich.

Instrument and Model: Nuclear Magnetic Resonance Instrument, Bruker-400 mhz UltraShield NMR; High Pressure Liquid Chromatography, Agilent-1100 Series HPLC; Mass Spectrometer, Waters ZMD 2000 MC365 Mass Spectrometer

### Example 1

This example is utilized to illustrate the preparation of compound 1: 2,6-diisopropylphenyl 2-(1-(aminomethyl)cyclohexyl)acetate hydrochloride.

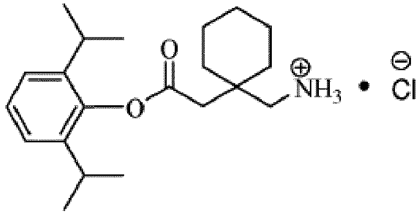

Gabapentin (5.14 g, 30 mmol, 1 equiv) was suspended in THF/water (1:1, 180 ml) with stirring and cooled to 0°C. NaHCO₃ (7.56 g, 90 mmol, 3 equiv) and Boc₂O (7.6 mL, 33 mmol, 1.1 equiv) were added sequentially, and the mixture was stirred at room temperature overnight. The reaction solution was extracted with diethyl ether (3×100 mL); the aqueous layer was acidified to pH 7 by carefully adding KHSO₄ (10%) at 0°C; the aqueous phases were combined and extracted with dichloromethane (3×100 mL); the two organic phases were combined and dried with Na₂SO₄, the solvent was removed under reduced pressure. Boc-gabapentin (7.4 g, 88%) was obtained as a white solid.

Boc-gabapentin (5.7 g, 21 mmol, 1 equiv) obtained above and propofol (3.9 mL, 21 mmol, 1 equiv) were dissolved in dichloromethane (100 mL), and 4-dimethylaminopyridine (164 mg, 7% mol) was added. A mixed solution of N,N'-dicyclohexylcarbodiimide (4.3 g, 21 mmol, 1 equiv) dissolved in dichloromethane (20 mL) was slowly added to the above mixture at 0 °C, and then stirred at 20 °C overnight. The next day the reaction solution was filtered to remove the precipitate, and the filtrate was depressurized to remove solvents, the coupled product was purified by silica gel chromatography, eluted with 8% ethyl acetate in hexane (6.0 g, 66%).

The purified coupling compound obtained above (6.0 g, 13.9 mmol, 1 equiv) was dissolved in 2N hydrogen chloride in diethyl ether (70 ml, 10 equiv), the solution was stirred for 2 days, and then the solvent was removed to give a white solid (5.1 g, 100%). The final product was then recrystallized from diethyl ether.

¹H NMR(*chloroform-d₁*)δ:8.54(br.s.,3H)*,* 7.13-7.25(m,3H), 3.15(br.s.,2H), 2.95(s,2H), 2.80-2.90(m , 2H), 1.47-1.70 (m, 10H), 1.19 (d, *J*=7.0Hz, 12H); MS (*m*/*z*): calcd for C₂₁H₃₄ClNO₂, 367.96; found, [M-Cl]⁺, 331.87.

The solubility of the compound 1 in water is: 18 mg/ml; LogP: 1.10.

### Example 2

This example is utilized to illustrate the preparation of compound 2: 4-iodo-2,6-diisopropylphenyl 2-(1-(aminomethyl)cyclohexyl)acetate hydrochloride.

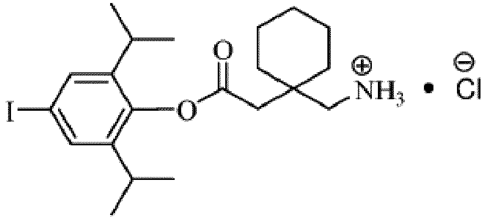

Propofol (3.43 mL, 18.5 mmol, 1 equiv) was dissolved in methanol (50 mL), sodium iodide (2.77 g, 18.5 mmol, 1 equiv) and sodium hydroxide (0.74 g, 18.5 mmol, 1 equiv) were added. The reaction is preferably carried out with stirring at 0°C. Sodium hypochlorite (35 mL, 4%) was added slowly at 0°C and the reaction was stirred for 1 hour, then quenched with sodium thiosulfate solution (20 mL, 10%). The pH of the reaction solution was adjusted to neutrality with 5% hydrochloric acid, and the crude product was obtained by extraction with diethyl ether. Purifying and eluting by silica gel chromatography with hexane solution of 3% ethyl acetate. 4-iodopropofol was obtained as a brown oil (2.4 g, 43%).

Gabapentin (5.14 g, 30 mmol, 1 equiv) was suspended in THF/water (1:1, 180 ml) with stirring and cooled to 0°C. NaHCO₃ (7.56 g, 90 mmol, 3 equiv) and Boc₂O (7.6 mL, 33 mmol, 1.1 equiv) were added sequentially, and the mixture was stirred at room temperature overnight. The reaction solution was extracted with diethyl ether (3×100 mL); the aqueous phase was adjusted to pH 7 by carefully adding KHSO₄ (10%) at 0°C; the aqueous phases were combined and extracted with dichloromethane (3×100 mL); the two organic phases were combined and dried with Na₂SO₄, the solvent was removed under reduced pressure. Boc-gabapentin (7.4 g, 88%) was obtained as a white solid.

Boc-gabapentin (2.0 g, 7.5 mmol, 1 equiv) and 4-iodopropofol (2.3 mL, 7.5 mmol, 1 equiv) were dissolved in dichloromethane (20 mL) and 4-dimethylaminopyridine ( 60 mg, 7% mol) was added. A mixed solution of N,N'-dicyclohexylcarbodiimide (1.54 g, 7.5 mmol, 1 equiv) dissolved in dichloromethane (10 mL) was slowly added to the mixture at 0°C and stirred at 20°C overnight. The next day the reaction solution was filtered to remove the precipitate, and the filtrate was depressurized to remove solvents. Purifying and eluting the coupled product by silica gel chromatography with 8% ethyl acetate in hexanes (2.74 g, 66%).

The purified coupling compound obtained above (2.74 g, 5 mmol, 1 equiv.) was dissolved in 2N hydrogen chloride in diethyl ether. (25 ml, 10 equiv.), stirred for 2 days, and then the solvent was removed and finally obtained a white solid (2.4 g, 100 %).

¹H NMR(*chloroform-d₁*)δ: 8.46(br.s.,3H), 7.44(s,2H), 3.13(br.s.,2H), 2.94(s,2H), 2.76-2.88(m,2H), 1.45-1.68 (m, 10H), 1.16 (d, *J*=7.0Hz, 12H); MS (*m*/*z*): calcd for C₂₁H₃₃ClINO₂, 493.85; found, [M-Cl]⁺, 457.71.

The solubility of the compound 2 in water is: 13 mg/ml; LogP: 1.66.

### Example 3

This example is utilized to illustrate the preparation methord of compound 3: 2-(1-(((2,6-diisopropylphenoxy)carbonyl)methyl)cyclohexyl)acetic acid.

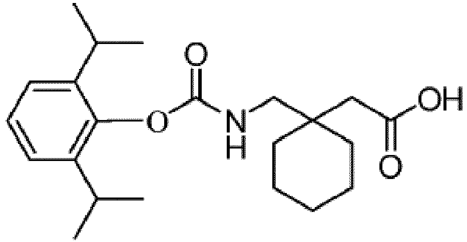

Gabapentin (0.42 g, 2.45 mmol, 1 equiv) was suspended in dichloromethane (20 mL) and cooled to 15°C, added diisopropylethylamine, and then added chlorotrimethylsilane (0.62 mL, 4.9 mmol, 2 equiv), the solution (Solution A) was stirred for 2 hours. In another reaction vessel, propofol (0.48 mL, 2.56 mmol, 1.04 equiv) was dissolved in dichloromethane (10 mL) at 0 °C , added trichloromethyl carbonate (triphosgene) (0.37 g, 1.28 mmol, 0.5 equiv), and added diisopropylethylamine (1.34 mL, 3.8 mmol, 3 equiv), stirred at 20°C for 12 hours, evaporated to near dryness under reduced pressure at 25 °C, and redissolved in dichloromethane ( 20 mL) (solution B). The solution A was slowly added dropwise to the solution B, stirred at 25 °C for 12 hours. The reaction was extracted with dichloromethane to obtain the crude product, which was further purified by column chromatography and eluted with 20% ethyl acetate in hexanes. 2-(1-(((2,6-diisopropylphenoxy)carbonyl)methyl)cyclohexyl)acetic acid (0.64 g, 59%) was obtained as a white solid.

¹H NMR(*chloroform-d₁*)δ:7.13-7.18(m,3H), 5.61(t,*J*=6.7Hz,1H), 3.37(d,*J*=6.8Hz,2H), 2.97-3.1(m,2H), 2.39(s,2H), 1.50-1.63(m,4H), 1.40-1.49(m,6H), 1.22(d,*J*=6.8Hz,12H); MS(*m*/*z*):calcd for C₂₂H₃₃NO₄, 375.5 ;found, [M+1]⁺,375.94.

The solubility of the compound 3 in water is: 0.08 mg/ml; LogP: 2.38.

### Example 4

This example is utilized to illustrate the preparation of compound 4: 2-(1-(((4-iodo -2,6-diisopropylphenoxy)carbonyl)methyl)cyclohexyl)acetic acid.

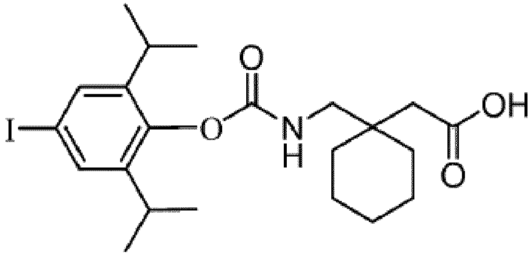

Gabapentin (0.42 g, 2.45 mmol, 1 equiv) was suspended in dichloromethane (20 mL) and cooled to below 15°C, added diisopropylethylamine, and then added chlorotrimethylsilane (0.62 mL, 4.9 mmol, 2 equiv) , stirred for 2 hours (solution A). In another reaction vessel, 4-iodopropofol (0.48 mL, 2.56 mmol, 1.04 equiv) was dissolved in dichloromethane (10 mL) at 0 °C, added trichloromethyl carbonate (triphosgene) ( 0.37 g, 1.28 mmol, 0.5 equiv), and added diisopropylethylamine (1.34 mL, 3.8 mmol, 3 equiv), stirred at 20°C for 12 hours, the reaction solution was evaporated to dryness under reduced pressure at 25 °C, and then redissolved in dichloromethane (20 mL) (solution B). Solution A was slowly added dropwise to mixture B and stirred at 25 °C for 12 hours. The reaction was extracted with dichloromethane to obtain the crude product, which was further purified by column chromatography and eluted with 20% ethyl acetate in hexanes. 2-(1-(((4-iodo -2,6-diisopropylphenoxy)carbonyl)methyl)cyclohexyl)acetic acid (0.88 g, 72%) was obtained as a white solid.

¹H NMR(*chloroform-d₁*)δ: 7.43(s, 2H), 5.64(t, *J*=6.7Hz, 1H), 3.36(d, *J*=7.2Hz, 2H), 2.89-3.02(m, 2H), 2.38 (s, 2H), 1.49-1.70 (m, 10H), 1.19 (d, *J*=6.8Hz, 12H); MS (*m*/*z*): calcd for C₂₂H₃₂INO₄, 501.4; found, [M+1]⁺, 501.73 .

The solubility of the compound 4 in water is: 0.04 mg/ml; LogP: 3.52.

### Example 5

This example is utilized to illustrate the preparation of compound 5: 2,6-diisopropylphenyl 2-(1-((dimethylamino)methyl)cyclohexyl)acetate hydrochloride.

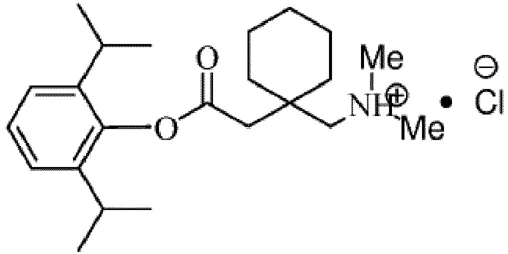

Gabapentin (10 g, 58.34 mmol, 1 equiv) was dissolved in 37% aqueous formaldehyde (13 mL, 175.02 mmol, 3 equiv), 95% formic acid (9.44 mL, 233.36 mmol, 4 equiv) was added, and the reaction mixture was stirred and refluxed at 90 °C for 5 hours. After cooling to room temperature, the reaction solution was concentrated under reduced pressure, mixed with concentrated hydrochloric acid (37%) (6 mL), and stirred for 1 hour. Recrystallization in acetone and ether (4.1 g, 30%) to obtain N,N'-dimethylgabapentin.

N,N'-dimethylgabapentin(1.9 g, 8 mmol, 1 equiv) was dissolved in a mixed solvent of dichloromethane (15 mL) and oxalyl chloride (1.7 mL, 19.5 mmol, 2.4 equiv), and a drop of dimethylformaldehyde was added . The mixture was stirred in an ice bath for 30 minutes, then stirring at 0 to 20 °C for 5 hours to obtain the acid chloride product of N,N'-dimethylgabapentin.

The resulting pale yellow solid was redissolved in dichloromethane (15 mL) in an ice bath, added pyridine (1.9 mL, 24.2 mmol, equiv) slowly, and then added propofol (1.1 mL, 6.45 mmol, 0.8 equiv) slowly, stirred at 20°C for 12h. The reaction solution was then washed with 1N aqueous HCl, and extracted with dichloromethane to obtain a crude product. The crude product was subjected to silica gel chromatography, eluting with 5% methanol in dichloromethane. Recrystallization in a mixed solvent of acetone and hexane (1.0 g, 39%) to obtain the final product.

¹H NMR(*chloroform-d₁*)δ:11.50(s, 1H), 7.08-7.13(m, 3H), 3.46-3.58(m, 2H), 3.30-3.41(m, 4H), 2.79-2.91(m, 6H), 1.16-1.22 (m, 10H), 1.12 (d, *J*=6.9Hz, 12H); MS (*m*/*z*): calcd for C₂₃H₃₈ClNO₂, 396.01; found, [M-Cl]⁺, 359.84.

The solubility of the compound 5 in water is: 150 mg/ml; LogP: 1.37.

### Example 6

This example is utilized to illustrate the preparation of compound 6: (1-(2-(2,6-diisopropylphenoxy)-2-oxoethyl)cyclohexyl)-N,N,N-tri-methylmethanaminium iodide.

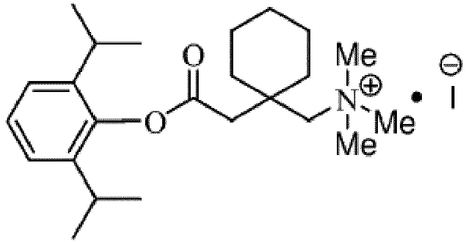

Compound 5 prepared in Example 5: 2,6-diisopropylphenyl 2-(1-((dimethylamino)methyl)cyclohexyl)acetate hydrochloride (0.8 g, 2 mmol, 1 equiv. ) was dissolved in water, adjusted to pH 8 with aqueous sodium carbonate (10%, 40 mL), and the free amine was extracted from the aqueous layer with dichloromethane. After removing the solvent under reduced pressure, iodomethane (5 mL) was added, stirred for 12 hours. Recrystallization with diethyl ether (0.9 g, 89%) to obtain the final product.

¹H NMR(*chloroform-d₁*)δ: 7.16-7.14(m, 3H), 3.87(s, 2H), 3.59(br.s., 2H), 3.53(s, 9H), 3.58(s, 4H), 3.10 (s, 2H), 2.69-2.80 (m, 2H), 1.17-1.20 (m, 10H), 1.10-1.15 (m, 12H); MS (*m*/*z*): calcd for C₂₄H₄₀INO₂, 501.48; found, [M-I ]⁺,373.97.

The solubility of the compound 6 in water is: 0.9 mg/ml; LogP: 1.54.

### Experimental Example 1

This experimental example is utilized to illustrate the effect of compound 5 prepared in Example 5 on nitroglycerin-induced migraine mouse model.

### Experimental animals and groups, sources

48 ICR mice weigt 18-22g, half male and half female; purchased from Qinglongshan Animal Breeding Farm, Jiangning District, Nanjing City, certificate: SCXK (Su) 2017-0001; rearing conditions: room temperature 18-20 °C, same sex rearing in separate cages , 12 hours of light, regular ventilation, conventional full-price solid feed feeding, bedding changed every two days; 48 mice were randomly divided into 6 groups (blank control group, model group, positive drug group, low-dose group of test drug, medium-dose group of test drug, high-dose group of test drug), 8 animals (4♀4) in each group, marked No. 1-8 with picric acid respectively.

### Experimental Drugs and Formulations

Solvent: 0.5% CMC-Na aqueous solution; positive drug: flunarizine hydrochloride (sibelin) capsules (specification 5 mg), take the contents in a mortar, add 100 ml of 0.5% CMC-Na solution while grinding, placed in EP tubes for standby; Test drug (compound 5): dissolve into 5, 10, 20 mg/ml solutions with distilled water respectively, and placed in EP tubes for standby; Modeling drug (nitroglycerin): take 2 nitroglycerin tablets (specification 5mg), crushed into powder, placed in EP tubes, added 500 µl of propylene glycol with a pipette and shaken well, standing still for 5 minutes, added 9.5 ml of normal saline, shaken well and standing still overnight, take the supernatant (containing nitroglycerin) 1mg/ml) for use.

### Modeling method

Except for the first group (blank control group), animals in other groups were subcutaneously injected with 10 mg/kg (0.2 ml/20 g) of nitroglycerin back-up solution 1 h after each administration (modeling once every other day on the 1st, 3rd, and 5th days) .

### Administration method, dosage

The first group was the blank control group, which was given 0.5% CMC-Na 0.2ml/20g by gavage; the second group was the model group, which was given 0.5% CMC-Na by gavage; the third group was the positive drug group, which was given by gavage Flunarizine hydrochloride (sibilin) 1 mg/kg; the fourth group was the low-dose group of test drug, which was given 100 mg/kg of compound 5 by gavage; the fifth group was the medium-dose group of test drug, which was given 200 mg/kg of compound 5 by gavage; the sixth group was the high-dose group of test drug, which was given 400 mg/kg of compound 5 by gavage. Dosing every other day on days 1, 3, and 5.

### Detection method

On the 5th day, the behavioral indicators of the mice were counted immediately after the modeling, that is, the number of scratches of each mouse was counted in three periods of 1-30 min, 30-60 min, and 60-90 min after modeling, and record when the mouse first exhibited head scratching behavior; measure the licking time of each mouse's hind foot using a hot plate instrument 45 minutes after modeling and record it.

### Observation Indicator

In behavioral studies, mice scratching their heads for more than 5 times or scratching their faces with forelimbs for more than 5 times were identified as migraine-related behaviors caused by modeling, with a record of scratching their heads once; in the hot plate method, mice lifted their hind limbs and licked their hind feet as the observation indicator, record the time immediately.

### Results

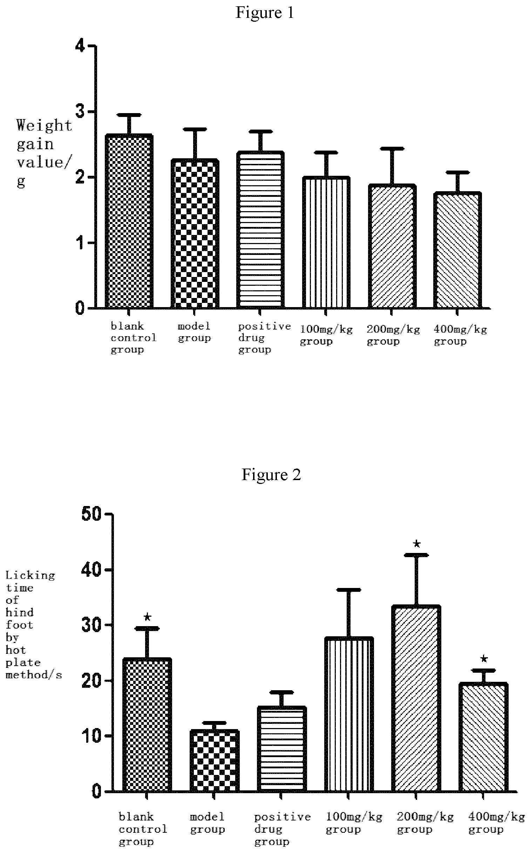

As shown in Figures 1-3.

### Conclusions

Regarding the weight of the mice, the data showed that the drug had no significant effect on them. Statistically, with the blank group as the reference, there was no significant difference in weight gain in all other groups (p>0.05).

The results of pain threshold and behavioral head scratching according to the hot plate test showed that the nitroglycerin-induced migraine model in mice was successful, and the test drug compound 5 could effectively alleviate nitroglycerin-induced migraine in mice. The statistical results showed that, taking the model group as the reference, there was a significant difference (p< 0.05) in the time spent licking the hind feet of the blank group mice, and the total number of head scratching was extremely significant (p< 0.01); there was a significant difference in licking time between the positive drug and the middle and high dose groups of the test drug (p< 0.05), the total number of head scratching times between the positive drug and the low and medium dose groups of the test drug was extremely significant (p< 0.01), while the high dose group of the test drug had a significant difference (p< 0.05).

### Experimental Example 2

This experimental example is utilized to illustrate the effect of compound 5 prepared in Example 5 on the lipopolysaccharide-induced depression mouse model.

Experimental animals and groups, sources

Same as Experimental Example 1.

### Investigational Drugs and Formulations

Positive drug: fluoxetine hydrochloride capsule (specification 20mg), take the content in a mortar, add 40ml of 0.5% CMC-Na solution while grinding, to a suspended state, set EP tube for use; test drug (compound 5 ): dissolve into 5, 10, and 20 mg/ml solutions in distilled water, respectively, and put them in EP tubes for later use; modeling drug (LPS): take a frozen lipopolysaccharide PBS solution (1.2 mg: 1.2 ml), and after thawing Use a pipette to take 1ml into the EP tube, replace with a new pipette tip and pipette 9ml of normal saline into the EP tube, shake well for use.

### Modeling method

Except for the first group (blank control group), other groups of animals were intraperitoneally injected with LPS 2h before the last administration on the 7th day to the mice in the model group, the positive drug group, and the low, middle and high dose groups of the test drug.

### Administration method, dosage

The first group was the blank control group, which was given 0.5% CMC-Na 0.2ml/20g by gavage; the second group was the model group, which was given 0.5% CMC-Na by gavage; the third group was the positive drug group, which was given by gavage Flunarizine hydrochloride (sibilin) 1 mg/kg; the fourth group was the low-dose group of the test drug, which was given 100 mg/kg of compound 5 by gavage; the fifth group was the medium-dose group of the test drug, which was given 200 mg/kg by gavage. kg of compound 5; the sixth group was the test drug high-dose group, and 400 mg/kg of compound 5 was administered by gavage. The drug was administered for one week, and the second to sixth groups were intraperitoneally injected with 1 mg/kg (0.2 ml/20 g) of LPS on the seventh day to establish a model.

### Spontaneous activity detection

On the 7th day, before the last administration and modeling, the effect of the drug on the spontaneous activity of the mice should be investigated, and it is excluded that the drug increases the spontaneous activity of the mice and interferes with the immobility time in the forced swimming experiment. The total migration distance of each mouse in the open field (50 × 50 × 50 cm) within 5 min was calculated using the open field experimental instrument and related computer software. Before the data collection, each mouse was given 5min to familiarize with the open field environment, after each mouse was tested, its excrement was removed and the odor was eliminated with an alcohol spray bottle.

### Forced Swimming Test (FST)

4h after intraperitoneal injection of LPS, the behavioral indicators of the mice were collected with a forced swimming test instrument and related computer software, and the swimming conditions of each mouse in the cylinder were observed for 6 minutes, and the mice were recorded within 4 minutes (ie, 2-6 minutes) of immobility time. Because the air temperature is low, it is appropriate to control the water temperature by adding hot water to the cylinder.

### Observation Indicator

In the spontaneous activity, the behavioral index selects the total migration distance of mice in the open field within 5 minutes,
by tracking the movement trajectory of mice through monitoring devices, and using computer software, the total distance of movement can be directly obtained; in the FST experiment, the mice were placed in a into a transparent cylinder filled with warm water (about 25°C) for 6 minutes, the mice will experience struggling, swimming and floating behaviors, the time when the mice stop struggling to float on the water surface is identified as a state of despair caused by depression, by using monitoring equipment and computer software to statistically analyze the state of mice after 4 minutes, the total "Immobility time" can be directly obtained.

### Results

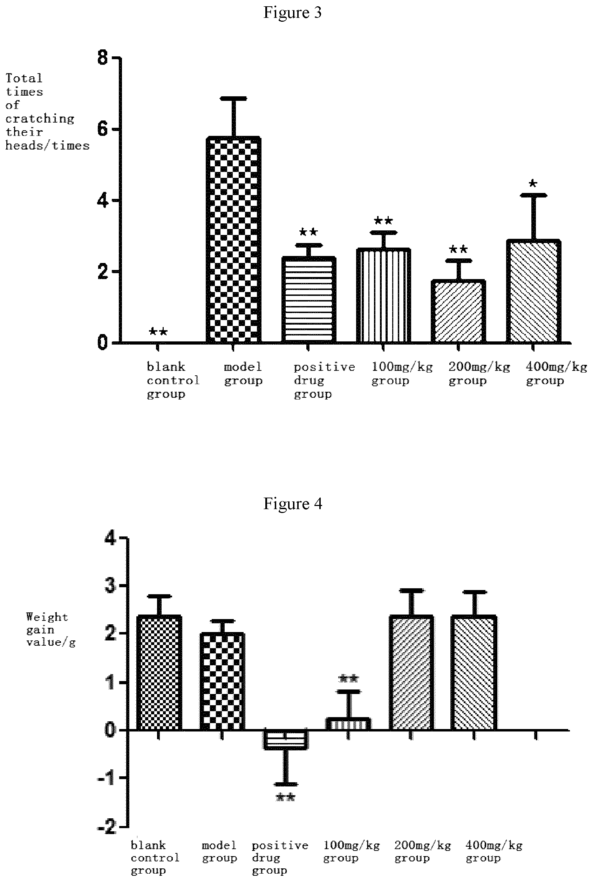

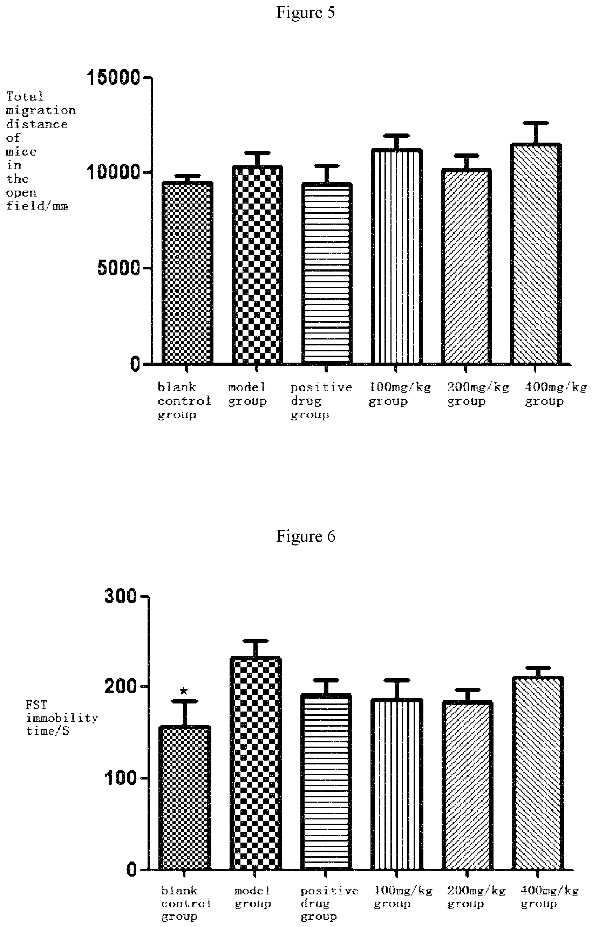

As shown in Figures 4-6.

### Conclusions

The results of the mouse body weight investigation showed that the positive drug and the test drug had no significant effect on the body weight of the mice. However, it was found in statistics that with the blank group as the reference, there was a significant difference in weight gain between the positive drug group and the low-dose test drug group (p<0.01).

For spontaneous activity, the positive drug and the test drug had no significant effect on the spontaneous activity of mice. In statistics, the blank group was utilized as the reference, and there was no significant difference in the total migration distance of mice in other groups within 5 minutes (p>0.05).

For the depression index - FST immobility time, the acute depression model was successfully replicated by the LPS method, and the positive drug and the test drug had a certain alleviation effect on the depressive behavior cautilized by LPS (no statistical difference was found); statistical taking the model group as the reference, the immobility time of the blank group was significantly different (p<0.05), and the immobility time of the positive drug and test drug groups was not significantly different (p>0.05).

### Experimental Example 3

This experimental example is utilized to illustrate the anticonvulsant effect of compound 5 prepared in Example 5 on mice induced by electrical stimulation.

### Source of experimental animals

The source is the same as that of Experimental Example 1.

### Animal screening and grouping

① Divide 40 mice (20♀20) into 5 groups at random, with 8 mice (4♀4) in each group, and label No. 1 to No. 8 with picric acid respectively; (2) All mice were tested according to the order of groups and labels electrical stimulation, the initial voltage was 40V, the voltage was increased by 2.5V each time, the time interval between two electrical stimulations for the same mouse was 20 min, the threshold voltage value of convulsion and the time from convulsion to complete recovery were recorded, and the dead mice were discarded, qualified mice were screened; ③ After screening, 7 mice (2♀5) in the 40 mice could not recover after convulsions and died, and the remaining 33 qualified mice (18♀15); ④From standby 7 mice were screened out as a supplement, and the number of the dead mice in step (2) was marked with picric acid and added to the corresponding groups, so that a total of 40 (20♀20) qualified mice in 5 groups were finally obtained. , all mice were weighed and recorded.

### Experimental Drugs and Formulations

Positive drug: sodium valproate (specification 0.2g), take 1 tablet in a mortar, add 20ml of 0.5% CMC-Na solution while grinding, to a suspended state, set EP tube for use; test drug (compound 5): Dissolve in distilled water to 5, 10, and 20 mg/ml solutions, respectively, and set EP tubes for later use.

### Modeling method

Electrical stimulation was performed 1.5 hours after the last administration, and each mouse was given the threshold voltage measured before to observe the experimental phenomenon and record whether there was convulsions and death occurred.

### Administration method, dosage

The first group was the blank control group, which was given 0.4ml/20g of 0.5% CMC-Na by gavage; the second group was the positive drug control group, which was given sodium valproate 200mg/kg by gavage; the third group was the test drug the low-dose group, which was given 100 mg/kg of compound 5 by gavage; the fourth group was the test drug medium-dose group, which was given 200 mg/kg of compound 5 by gavage; the fifth group was the test drug high-dose group, which was given 400 mg/kg of compound 5 by gavage.

### Observation Indicator

1.5h after the last administration, electrical stimulation was performed according to the threshold voltage measured before each mouse, the experimental phenomenon was observed, and the occurrence of convulsions and death were recorded. If convulsion occurs, record the time of complete recovery; if no convulsion occurs, continue to increase the voltage with a gradient of 2.5V to measure the current threshold voltage value and recovery time.

Strictly follow the 5 periods of convulsion in mice, namely, latency period, rigid flexion period, hindlimb extension period, clonic period and recovery period to judge whether the mice have convulsions after electrical stimulation, and the phenomenon of death can not be recovered after convulsions.

### Results

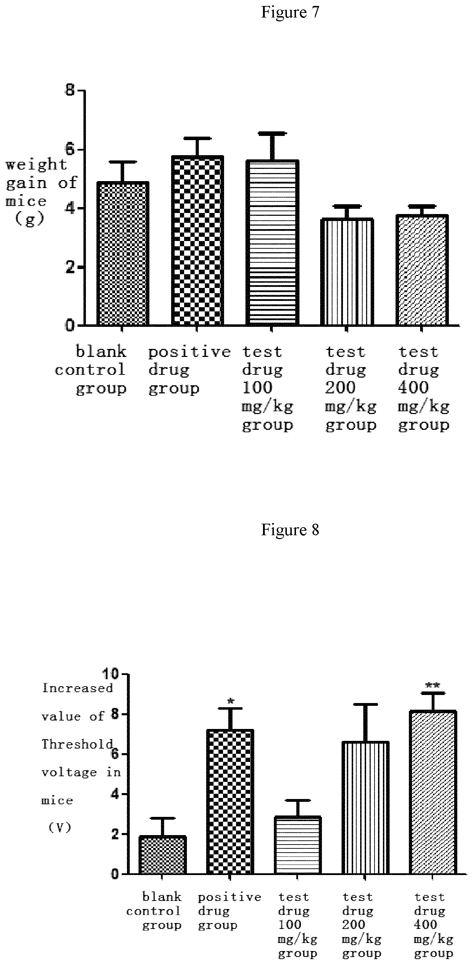

As shown in Figures 7-8.

### Conclusions

Compared with the blank group, there was no difference in the effect of three dose groups of positive drug and test drug on the weight gain of mice (p>0.05). Compared with the blank group, the positive drug group can significantly improve the anti-electrical convulsion ability of mice (p<0.05); the test drug 100 and 200 mg/kg dose groups have no significant effect on the anti-electrical convulsion ability of mice (p>0.05); The 400mg/kg dose group could significantly improve the anti-electrical convulsion ability of mice (p<0.01).

### Experimental Example 4

This experimental example utilized the hot plate method to investigate the analgesic effect of compound 1 prepared in Example 1 on mice after intravenous administration.

### Experimental animals and groups, sources

20 ICR mice, 18-22g, half male and half male, were purchased from the animal room of Xi'an Jiaotong University School of Medicine; rearing conditions: room temperature 18-20°C, same sex rearing in separate cages, 12-hour light, regular ventilation, conventional full-price solid feed feeding, bedding changed every two days; 20 mice were randomly divided into 2 groups (low-dose test drug group, test drug high-dose group) with 10 mice in each group (5 ♀5).

All the mice participating in the experiment were tested for hot plate pain threshold before the experiment, which proved that the mice participating in the experiment were qualified.

### Instruments

PL-200 Hot Sting Pain Meter (Chengdu Taimeng Technology Co., Ltd.)

### Experimental Drugs and Formulations

An appropriate amount of compound 1 was accurately weighed and dissolved in 0.9% physiological saline to prepare a concentration of 5 mg/ml (dissolved state is good, basically transparent), which was used as a mother solution. During the test, according to the test requirements, diluted with normal saline to an appropriate concentration.

### Administration method, dosage

The first group was the low-dose test drug group, and the test drug was given 10 mg/kg by tail vein injection; the second group was the test drug high-dose group, and the test drug was given 15 mg/kg by tail vein injection.

### Experiment method

The mice were placed in a hot plate apparatus at a temperature of 55±0.5°C, and the pain thresholds of the mice were measured and recorded before administration, 30 minutes after administration, 60 minutes after administration, and 120 minutes after administration.

The test results are shown in Table 1 below.

**Table 1. Effect of Compound 1 Hot Plate Method on Pain Domain in ICR Mice ( X̅±SD)**

| Dose (mg/kg) | Pain threshold before administration (s) | Pain threshold at different times after administration (s) | | |
|---|---|---|---|---|
| | | 30min | 60min | 120min |
| 10 | 8.92±3.05 | 17.09±6.05^{∗∗} | 16.96±6.13^{∗∗} | 12.36±5.75^{∗} |
| 15 | 10.90±3.72 | 18.88±6.06^{∗} | 13.49±6.53 | 6.47±5.40 |

| | | | | |
|---|---|---|---|---|
| Note: ^{∗}p<0.05 compared with before administration; ^{∗∗}p<0.01 | | | | |

### Conclusions

Compound 1 has a good analgesic effect on mice in the hot plate test, and the dose of 10 mg/kg has the best analgesic effect, in terms of duration of action, there is a good analgesic effect within 30-60 minutes after injection.

### Experimental Example 5

This experimental example utilized the hot plate method to investigate the analgesic effect of compound 1 prepared in Example 1 on mice after intragastric administration.

### Experimental animals and groups, sources

Same as Experimental Example 1.

All the mice participating in the experiment were subjected to hot plate pain threshold detection before the experiment, and qualified mice with pain threshold of 10-40s were screened.

### Instruments

### Chengdu Taimeng Intelligent Hot Plate Instrument (Chengdu Taimeng Software Co., Ltd.)

### Experimental Drugs and Formulations

Accurately weigh an appropriate amount of compound 1, dissolve it in distilled water and prepare a mother liquor of 100 mg/ml, then dilute to 1.25 mg/ml (25 mg/kg), 2.5 mg/ml (50 mg/kg) with double distilled water respectively; accurately weigh an appropriate amount of the positive control drug gabapentin, dissolve in distilled water and prepare a 100 mg/ml mother solution, and then dilute to 10 mg/ml (200 mg/kg) with double distilled water. All medicines are prepared and utilized as needed.

### Administration method, dosage

40 only qualified mice were divided into 4 groups, half male and half female in each group. The first group is the blank group, no administration; the second group is the positive control (gabapentin) 25mg/kg group; the third group is the test drug 25mg/kg group; the fourth group is the test drug 50mg/kg group 4. All were administered by gavage.

### Experiment method

The mice were placed in a hot plate apparatus at a temperature of 55±0.5°C, and the pain thresholds of the mice were measured and recorded before administration and 60 min after administration.

The test results are shown in Table 2 below.

**Table 2. Effects of Compound 1 Hot Plate Method on Pain Threshold in Mice by gavage( X̅±SD)**

| Grouping | Dose (mg/kg) | Quantity (n) | Pain response time (s) | | Pain response time extension rate (%) |
|---|---|---|---|---|---|
| | | | Before administration | After administration | |
| Blank group | / | 10 | 21.8±7.1 | 23.1±11.5 | 3.9±6.6 |
| Gabapentin | 25 | 10 | 16.6±5.2 | 33.5±15.0^{∗} | 11 3.4±99. 1 ^{∗∗∗} |
| Test drug | 25 | 10 | 22.0±10.2 | 32.1±14.5^{∗} | 59.3±66.7^{∗∗} |
| | 50 | 10 | 20.0±5.9 | 40.2±17.9^{∗∗} | 100.0±91.1^{∗∗∗} |

| | | | | | |
|---|---|---|---|---|---|
| Note: Compared with blank group, ^{∗}p<0.05, ^{∗∗}p<0.01, ^{∗∗∗}p<0.001. | | | | | |

### Conclusions

Compound 1 can prolong the reaction time of hot plate pain, and its analgesic effect is comparable to that of the positive drug gabapentin.

### Experimental Example 6

This experimental example is utilized to illustrate the stability of compound 5 prepared in Example 5 in fresh and acidic rat plasma.
Experimental sample: compound 5 prepared in Example 5; 5% glucose (Xi'an Jingxi Shuanghe Pharmaceutical Co., Ltd.); rat plasma (source: Animal Center, Department of Medicine, Xi'an Jiaotong University)
Fresh plasma sample preparation:
a: Compound 5 prepared in Example 5 was dissolved in purified water to prepare solution A with a concentration of 10 mg/ml; 150 µl of solution A was added to 660 µl of preheated plasma (37°C) to obtain solution B;
b: Solution B (5 µmol/ml) was stirred in a 37°C water bath for a certain period of time (1min, 2min, 4min, 8min, 16min, 30min, 1h, 2h, 4h) and 50 µl was sampled at each time point;
c: 50 µl of sampled solution) was added to 950 µl of methanol (0.1% AcH) to precipitate plasma proteins, resulting in mixture C;
d: The mixture C was vortexed for 1 s, centrifuged at 12000 r for 3 min, and the supernatant was taken for testing.

Acidified plasma sample preparation:
a: Compound 5 prepared in Example 5 was dissolved in purified water to prepare solution A with a concentration of 10 mg/ml; 150 µl of solution A was added to 660 µl of pre-warmed plasma (37°C), and the pH was adjusted with 0.1% acetic acid to dissolve the sample clear to obtain solution B;
b: Solution B (5 µmol/ml) was stirred in a water bath at 37°C for a certain period of time (1min, 2min, 4min, 8min, 16min, 30min, 1h, 2h, 4h) and 50 µl was sampled at each time point;
c: 50 µl of the sampled solution was added to 950 µl methanol (0.1% AcH) to precipitate plasma proteins, resulting in mixture C.
d: The mixture C was vortexed for 1 s, centrifuged at 12000 r for 3 min, and the supernatant was taken for testing.

Liquid phase detection: take 10 µl of the supernatant after the above centrifugation into a sample for measurement.

**Table 3. Degradation test results of compound 5 in fresh rat plasma**

| Number | Sample Peak | Average | Propofol Peak | Average | Area Ratio (Propofol/Sample) |
|---|---|---|---|---|---|
| 1-1 (1min) | 203198951 | 203062103 | 26518146 | 30003338 | 0.147754 |
| | 202925254 | | 33488530 | | |
| 1-2 (2min) | 181805344 | 181706084 | 30185843 | 30444097 | 0.167546 |
| | 181606824 | | 30702351 | | |
| 1-3 (4min) | 161507165 | 161303805 | 27477210 | 27509271 | 0.170543 |
| | 161100445 | | 27541331 | | |
| 1-4 (8min) | 179024398 | 178769005 | 30835111 | 30981104 | 0.173302 |
| | 178513611 | | 31127096 | | |
| 1-5 (16min) | 189287319 | 189298224 | 32525178 | 32815652 | 0.173354 |
| | 189309128 | | 33106125 | | |
| 1-7 (1h) | 230597745 | 230407155 | 43810565 | 43571294 | 0.189106 |
| | 230216565 | | 43332023 | | |
| 1-8 (2h) | 261184871 | 260803581 | 50926502 | 51454807 | 0.197293 |
| | 260422291 | | 51983112 | | |
| 1-9 (4h) | 246770513 | 246523592 | 53721190 | 54249501 | 0.220058 |
| | 246276671 | | 54777811 | | |

**Table 4. Degradation test results of compound 5 in acidified plasma**

| Number | Sample Peak | Average | Propofol Peak | Average | Area Ratio (Propofol/Sample) |
|---|---|---|---|---|---|
| 2-1(1min) | 196891222 | 196752060 | 33786927 | 33910212 | 0.17235 |
| | 196612898 | | 34033497 | | |
| 2-2(2min) | 189254121 | 189140125 | 33679500 | 33755871 | 0.17847 |
| | 189026129 | | 33832241 | | |
| 2-3(4min) | 184558026 | 184526452 | 33909647 | 34069483 | 0.184632 |
| | 184494877 | | 34229319 | | |
| 2-4(8min) | 182496199 | 182379068 | 36705521 | 36897732 | 0.202313 |
| | 182261937 | | 37089943 | | |
| 2-5(16min) | 176486180 | 176475001 | 34262582 | 34666342 | 0.196438 |
| | 176463822 | | 35070102 | | |
| 2-6(30min) | 179574250 | 179508039 | 35311403 | 35340116 | 0.196872 |
| | 179441828 | | 35368828 | | |
| 2-7(1h) | 175097628 | 175087504 | 36583810 | 36564840 | 0.208838 |
| | 175077379 | | 36545869 | | |
| 2-8(2h) | 171502623 | 171193983 | 36992596 | 37504102 | 0.219074 |
| | 170885343 | | 38015608 | | |

### Conclusions:

According to the detection results of plasma samples, it can be seen that the test samples are not significantly degraded in fresh plasma and acidified plasma, indicating that the samples are relatively stable in fresh plasma and acidified plasma.

### Experimental Example 7

This experimental example utilized the hot plate method to investigate the analgesic effect of compound 2 prepared in Example 2 on mice after intravenous administration.

### Experimental animals and groups, sources

20 ICR mice, 18-22g, half male and half male, were purchased from the animal room of Xi'an Jiaotong University School of Medicine; rearing conditions: room temperature 18-20°C, same-sex cages, 12-hour light, regular ventilation, conventional full-price solid feed feeding, and the bedding was changed every two days; 20 mice were randomly divided into 2 groups (low-dose test drug group, test drug high-dose group), 10 mice in each group (5 ♀5).

All the mice participating in the experiment were tested for hot plate pain threshold before the experiment, which proved that the mice participating in the experiment were qualified.

### Instruments

PL-200 Hot Sting Pain Meter (Chengdu Taimeng Technology Co., Ltd.)

### Experimental Drugs and Formulations

An appropriate amount of compound 2 was accurately weighed and dissolved in 5% glucose water to prepare a concentration of 2 mg/ml (dissolved state is good, basically transparent), which was utilized as a mother solution. During the test, according to the test requirements, it was diluted with 5% glucose aqueous solution to an appropriate concentration.

### Administration method, dosage

The first group was the high-dose test drug group, and the test drug was given 10 mg/kg by tail vein injection; the second group was the low-dose test drug group, and the test drug was given 3 mg/kg by tail vein injection.

### Experiment method

The mice were placed in a hot plate apparatus at a temperature of 55±0.5°C, and the pain thresholds of the mice were measured and recorded before administration, 30 minutes after administration, 60 minutes after administration, and 120 minutes after administration.

The test results are shown in Table 5 below.

**Table 5. Effects of compound 2 hot plate method on pain domain in ICR mice ( X̅±SD)**

| Dose (mg/kg) | Pain threshold before administration (s) | Pain threshold at different times after administration (s) | | |
|---|---|---|---|---|
| | | 30 min | 60 min | 120 min |
| 10 | 13.18±3.33 | 18.10±5.96^{∗} | 13.98±6.78 | 14.18±6.90^{∗} |
| 3 | 11.33±4.08 | 12.41±3.67 | 10.16±3.38 | 6.47±5.40 |

| | | | | |
|---|---|---|---|---|
| Note: ^{∗}p<0. 05compared with before administration. | | | | |

### Conclusions:

Compound 2 showed good analgesic effect in mice in the hot plate test, and at 10 mg/kg, it performed well 30 minutes after injection.

### Experimental Example 8

This experiment utilized a mouse righting reflex test to investigate the sedative and hypnotic effects of compound 5 and its related compatibility.

**Experimental animals,** SPF Kunming mice, male, 20-30g, Laboratory Animal Center of Lanzhou University (certificate number: SCXK (Gan) 2018-0002), reared in separate cages, temperature, 22±1°C, humidity, 50%, automatic in the light-controlled 12h/12h light-dark cycle (light-on time 8:00h, light-off time 20:00h, light intensity ≈ 100lux) environment, free food and activities, adapted to the experimental environment for 5 days.

**Experimental drug,** compound 5; Gabapentin (A); Propofol (abbreviated as Pro or P, 10mg/ml dissolved in 10% DMSO); Diazepam (abbreviated as Dia) injection, specification: 2ml: 10mg, Tianjin Jinyao Pharmaceutical Industrial Co., Ltd., batch number 1903111; Pro and gabapentin (A) are mixed at a dosage of 1:1 (molar ratio) (dissolved in 10% DMSO). Grouping and administration, 42 Kunming mice were selected and divided into 6 groups with 7 mice in each group, as shown in Table 6 below, all of which were administered by intraperitoneal injection.

**Table 6: Grouping and Dosage**

| Grouping | Group | Dosage |
|---|---|---|
| Negative Control 1 | Saline group | 0.1ml/10g |
| Negative Control 2 | 10%DMSO group | 0.1ml/10g |
| Positive Control | Diazepam group (Dia group) | 5mg/kg |
| Test Drug 1 | Propofol group (Pro group) | 25mg/kg |
| Test Drug 2 | Gabapentin and Pro group (A1P1 group) | A and P (24 and 25 mg/kg, respectively) |
| Test Drug 3 | Compound Group 5 | 56mg/kg |

| | | |
|---|---|---|
| Note: Diazepam injection was diluted 10 times with normal saline, and the dosage was 0.1ml/10g, the same experiment 1, 2 and 3 groups were prepared separately, and the dosage was 0.1ml/10g. | | |

**Righting reflex experimental method,** the mice were weighed, and after intraperitoneal injection of drugs according to the corresponding dose, the following contents were recorded (if the animal's righting reflex did not disappear after 20 minutes of drug injection, it was regarded as no righting reflex disappearance): ①The time from the start of administration to the disappearance of the righting reflex of the mouse (the disappearance of the righting reflex lasted for more than 30s) is the latency of the disappearance of the righting reflex; (2) Record the time from the disappearance of the righting reflex to the recovery of the righting reflex (completed within 1 min for 3 righting reflexs is regarded as the righting reflex recovery); ③ Observe and record the activity of the animal.

**Statistical analysis,** the experimental data were analyzed by SPSS 19.0 statistical software, and all data were expressed as Means±SEM.

**The experimental results** are shown in Table 7 below. The righting reflex of the mice in the negative control group (Saline and DMSO) and A1P1 group did not disappear, and there was no significant change in the activity of the mice; the righting reflex did not disappear in the diazepam group (Dia) and compound 5 group mice, but the activity state was significantly reduced, the gait was very unstable, and the drug effect was significant; the righting reflex of 2 mice in the propofol group (Pro group) disappeared, and the rest of the activities were significantly reduced. The results showed that compound 5 had better sedative effect.

**Table 7: Statistics of righting reflex in mice after intraperitoneal injection (n=7, Mean±SEM)**

| Dosage and grouping | Disappearance time | Duration | Observation of activity and other phenomena |
|---|---|---|---|
| Saline group | The righting reflex does not disappear | - | No significant change in activity level |
| 10%DMSO group | The righting reflex does not disappear | - | No significant change in activity level |
| Compound 5 group | The righting reflex does not disappear | - | Reduced activity |
| Pro group | 172.28 s | 150.97 s | The righting reflex disappeared in 2 mice, while other activities decreased |
| A1P1 group | The righting reflex does not disappear | - | No significant decrease in activity |
| Dia group | The righting reflex does not disappear | - | Unstable gait and reduced activity |

### Experimental Example 9

This experiment utilized the mouse sugar preference test to investigate the antidepressant effect of compound 5 and its related compatibility.

**Experimental drug,** clomipramine (CLI), sigma company, batch number: C7291, 10mg/ml (10% DMSO); reserpine (Res), sigma company, batch number: 83580, 1mg/ml ( 10% DMSO);
Compound 5; Gabapentin (A); Propofol (abbreviated as Pro or P, 10 mg/ml in 10% DMSO); Pro and gabapentin were mixed at a 1:1 dosage (10% in DMSO).

### Laboratory animals, grouping and dosing

SPF adult male Kunming mice, 20-30g, Laboratory Animal Center of Lanzhou University, animal certificate number: SCXK (Gan) 2018-0002, reared in separate cages, temperature 22±1 °C, humidity 50%, 12 h/12 h light and dark cycle controlled by dynamic light (light-on time 8:00h, light-off time 20:00h, light intensity ≈ 100lux), animals could eat and move freely. Male Kunming mice were selected, with 6-10 mice in each group, as shown in Table 8 below, all of which were administered by intraperitoneal injection.

**Table 8: Grouping and Dosage**

| Grouping | Group | Dosage |
|---|---|---|
| Negative Control | Control group (DMSO group, n=9) | 0.1ml/20g |
| Negative Control | Reserpine, DMSO group (Res + DMSO group, n=10) | 6 mg/kg |
| Positive Control | Reserpine,Clomipramine group (Res + CLI group, n=7) | Res, 6 mg/kg |
| | | CLI, 50 mg/kg |
| Test Drug 1 | Reserpine, Propofol group (Res + Pro group, n=8) | Res, 6 mg/kg |
| | | Pro, 25 mg/kg |
| Test Drug 2 | Reserpine, Gabapentin and propofol (1: 1) group (Res + A1P1 group, n=6) | Res, 6 mg/kg |
| | | Gabapentin and Pro (24 and 25 mg/kg respectively) |
| Test Drug 3 | Reserpine, Compound 5 group (Res+ Compound 5 group, n=7) | Res, 6 mg/kg |
| | | Compound 5, 56 mg/kg |

| | | |
|---|---|---|
| Note: The preparation concentration of reserpine is 1.2mg/ml, the preparation concentration of clomipramine is 10mg/ml, the preparation concentration of gabapentin and pro is 4.8mg/ml and 5mg/ml respectively, the preparation concentration of compound 5 is 11.2mg/ml, the administration volumes are all 0.1ml/20g. | | |

**Sugar water preference experiment method:** Kunming mice were selected and divided into groups, before the experiment, the animals were trained to drink 2% sucrose water, on the first day of the experiment, DMSO or Res was injected at 8:00, and DMSO, Compound 5, Pro or CLI were injected at 9:00. (Refer to Table 8 for details), then place 1 bottle of 2% sucrose water and 1 bottle of ordinary drinking water, and exchange the positions of sugar water and ordinary drinking water every 12 hours; after the second intraperitoneal injection of DMSO, compound 5, Pro, A1P1 or CLI (Refer to Table 8 for details) at 9:00 on the second day, the animals were fasted for 24 hours; 1 bottle of 2% sucrose water and 1 bottle of ordinary drinking water were placed at 9:00 on the third day, and the animals were weighed after 2 hours to calculate the drinking water, the amount of sucrose water and the amount of ordinary water were calculated by the formula of sugar water preference = sugar water drinking amount/total drinking amount, and used to evaluate the depressive behavior of animals.

**Statistical analysis,** the experimental data were analyzed by SPSS 19.0 statistical software, and all data were expressed as Means±SEM.

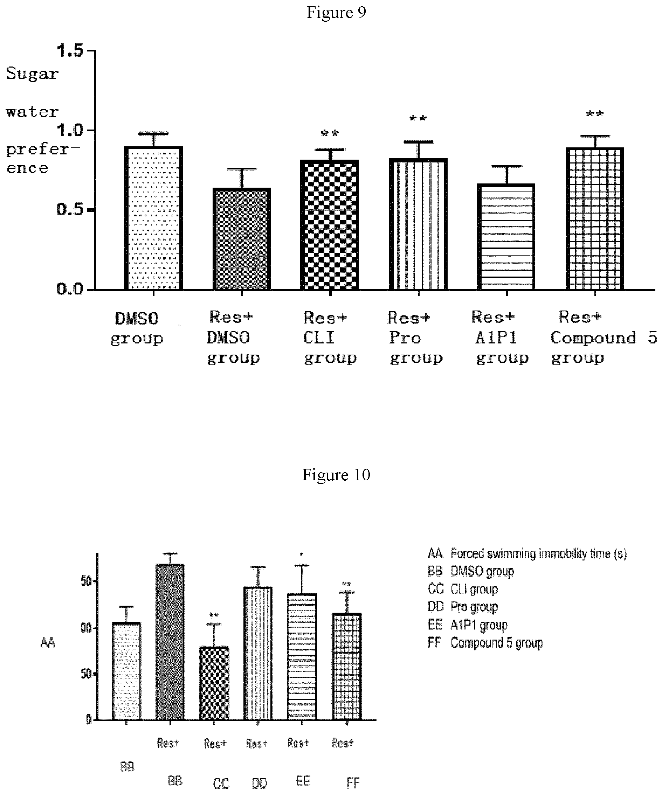

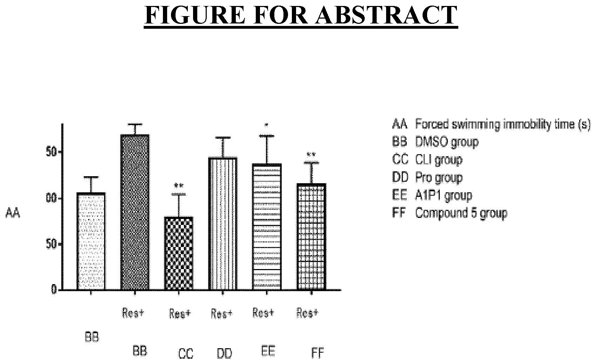

**Experimental results,** the specific results are shown in Table 9 below (shown in Figure 9), compared with the DMSO group, the mice in the Res+DMSO group have a significantly lower preference for sugar water, on the basis of administration in the Res group, after intraperitoneal injection of CLI, the preference for sugar water in mice significantly increased, indicating that the experimental process met the testing requirements. Compared with the Res+DMSO group, the mice in the other groups were given different drugs after injection of Res, among them, after administration of Pro and compound 5, the preference for sugar and water in the mice rose significantly and there were significant differences, and compound 5 had a significant antidepressant effect. , after administration of A1P1, there was no significant change in the preference for sugar water.

**Table 9: Statistical results of sugar water preference test**

| Number | Group | Sugar water preference |
|---|---|---|
| 1 | DMSO group | 0.901 ±0.026 |
| 2 | Res + DMSO group | 0.642±0.036 |
| 3 | Res + CLI group | 0.812±0.025^{∗∗} |
| 4 | Res + Pro group | 0.789±0.043^{∗∗} |
| 5 | Res+A1P1 group | 0.670 ± 0.043 |
| 6 | Res + Compound 5 group | 0.894±0.028^{∗∗} |

| | | |
|---|---|---|
| Note: Compared with the Res, ^{∗}P≤0.05; ^{∗∗}P≤0.01. | | |

### Experimental Example 10

This experiment utilized a forced swimming test in mice to investigate the antidepressant effect of compound 5 and its related compatibility.

### Experimental drug, animal, grouping and administration are same as Sugar water preference experiment method.

### Forced swimming test method

**Preparation of depression animal models**, acute intraperitoneal injection of 6 mg/kg Res can cause a continuous decrease in monoamine neurotransmitters in the brain for 1 week, and depression-like behaviors last for 72 hours, it is often utilized for the preparation of depression models (Huang et al., 2004). The experimental procedure was as follows: on the first day of the experiment, Res (6 mg/kg) or 10% DMSO was injected intraperitoneally at 8:00, DMSO, compound 5, A1P1, Pro or CLI were injected at 9:00, and the second injection of DMSO, compound 5, Pro, or CLI at 9:00 am on the second day, the forced swim test was completed between 9:00-12:00 on the third day.

**Forced swimming experiment process,** the forced swimming experiment is an experiment in which animals are placed in an unavoidable stressful environment to detect the despair state of animal behavior (Porsolt et al., 1978). Male Kunming mice were selected and randomly divided into groups. Conduct in a quiet and enclosed behavioral observation room, with red lights for indoor lighting (25lux). The experiment is divided into two stages, the first stage: adaptation experiment, 1 day before the test, 9:00-12:00h, put the animals into the forced swimming test equipment (plexiglass drum: diameter 20cm, height 25cm, water depth 12cm, water temperature 21-25 °C, the upper side CCD camera is connected to the computer to record) after 15 minutes of adaptation, take it out, dry the hair with a towel and put it back into the cage. The second stage: testing experiment, animals in each group were put into the forced swimming bucket for 5 minutes at the same time period, and the behavioral activities during the testing period were tracked and recorded by digital cameras and then analyzed. Immobility time: The animal floats on the water surface, with no movement of the limbs or slight movement of the hind limbs to keep the head above the water surface. Evaluate depression like behavior by detecting the despair state of animals based on their immobility time.

**Statistical analysis,** the experimental data were analyzed by SPSS 19.0 statistical software, and all data were expressed as Means±SEM. The data were analyzed by one-way ANOVA, and comparison between two groups using Fisher's least significant difference (LSD) test, and p<0.05 was considered statistically significant.

**Experimental results,** the specific results are shown in the following table 10 (shown in accompanying drawing 10):

**Table 10 Statistical results of forced swimming test**

| Number | Group | Forced swimming immobility time (s) |
|---|---|---|
| 1 | DMSO group | 106.72±16.49 |
| 2 | Res + DMSO group | 169.82±11.22 |
| 3 | Res + CLI group | 80.66±23.68^{∗∗} |
| 4 | Res + Pro group | 145.01±21.21 |
| 5 | Res+A1P1 group | 137.77±30.35^{∗} |
| 6 | Res + Compound 5 group | 116.29±22.23^{∗∗} |

| | | |
|---|---|---|
| Note: Compared with the Res, ^{∗}P≤0.05; ^{∗∗}P≤0.01. | | |

Compared with the DMSO group, the forced swimming immobility time of the mice in the Res+DMSO group was prolonged and significantly different, on the basis of the Res group administration, after intraperitoneal injection of CLI, the forced swimming immobility time of the depressed model mice was significantly shortened, the results showed that the experimental process meets the test requirements.

On the basis of the administration of Res group, different drugs were injected intraperitoneally. Compared with the Res+DMSO group, the forced immobility time of model mice after intraperitoneal injection of Pro or A1P1 was slightly shortened, and the forced immobility time of model mice after injection of compound 5 was slightly shortened; compared with the Res+Pro group, the forced immobility time of model mice was shortened and there was a significant difference after intraperitoneal injection of Res+compound 5; compared with the Res+A1P1 group, the model mice after intraperitoneal injection of Res+ compound 5, the forced immobility time of mice was shortened to a certain extent, but there was no significant difference. The results showed that compound 5 had significant antidepressant effect.

Although this invention has been described to a certain extent, it will be apparent that suitable changes in various conditions may be made without departing from the spirit and scope of the invention. It is to be understood that the invention is not limited to the embodiments described, but is to be included within the scope of the claims, which include equivalents for each of the elements described.

## Claims

1. A drug conjugate or a pharmaceutically acceptable salt thereof, **characterized in that**, the structure of the drug conjugate is shown in formula (I):
Dₚ-L-D_{g} (I)
wherein, D_{g} is gabapentin or its derivative; L is selected from carbonyl group or may not exist; Dₚ is selected from propofol or its derivative;
preferably, the structure of the pharmaceutically acceptable salt of the drug conjugate is shown in formula (II):
Dₚ-L-D_{g}•A (II)
wherein, A is a pharmaceutically acceptable acid anion, preferably is selected from one or more of the following: sulfate, phosphate, hydrochloride, hydrobromide, acetate, oxalate, citrate, succinic acid Salt, gluconate, tartrate, p-toluenesulfonate, benzenesulfonate, mesylate.

2. The drug conjugate or a pharmaceutically acceptable salt thereof according to claim 1, **characterized in that**, D_{g} is gabapentin or its derivative thereof as shown in formula (III): 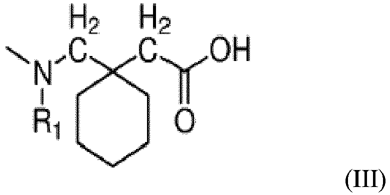 wherein, R₁ is a methyl group or a hydrogen atom.

3. The drug conjugate or a pharmaceutically acceptable salt thereof according to claim 1, **characterized in that**, D_{g} is gabapentin or its derivative thereof as shown in formula (IV): 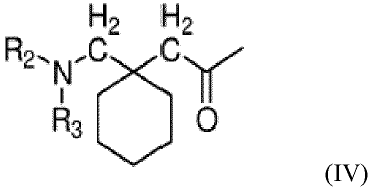 wherein, R₂ and R₃ are independently selected from methyl group or hydrogen atoms.

4. The drug conjugate or a pharmaceutically acceptable salt thereof according to claim 1, **characterized in that**, D_{g} is a derivative of gabapentin represented by formula (V): 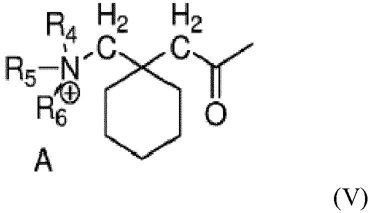 wherein, R₄, R₅ and R₆ are independently selected from methyl group or hydrogen atoms.

5. The drug conjugate or a pharmaceutically acceptable salt thereof according to any one of claims 1 to 4, **characterized in that**, Dₚ is propofol or its derivative thereof as shown in formula (VI): 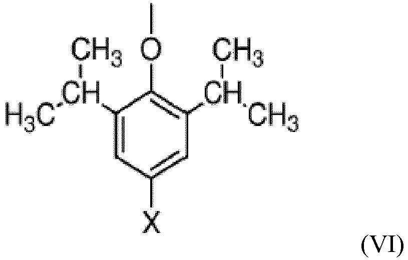 wherein, X is a halogen atom or a hydrogen atom.

6. The preparation method of a drug conjugate or a pharmaceutically acceptable salt thereof according to any one of claims 1 to 5, **characterized in that**, the method comprises the following steps:
the drug conjugate is prepared by suspending Dp and Dg in an organic solvent;
preferably, the organic solvent is selected from one or more of the following: dichloromethane, tetrahydrofuran/water, diethyl ether, methanol, ethanol, isopropanol, benzene, toluene; preferably dichloromethane and/or tetrahydrofuran/water.

7. The method according to claim 6, **characterized in that**, the method comprises the following steps: preparing the derivative D_{g} of gabapentin as described in formula (VII): 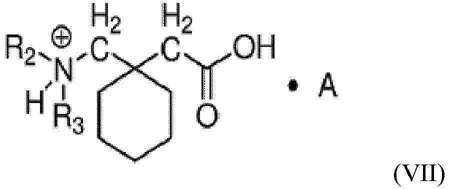
preferably, the steps comprises: dissolving gabapentin in an aldehyde solution, and adding a reducing agent to prepare a gabapentin derivative;
more preferably, the aldehyde solution is an aqueous formaldehyde solution, and the amount of the aldehyde is 1-5 molar equivalents, preferably 3 molar equivalents; and/or the reducing agent is formic acid, and the amount of the reducing agent is 1-5 molar equivalents, preferably is 4 molar equivalents;
further preferably, the reaction temperature is 60-90 °C; and/or the reaction time is 3-12 hours.

8. The method according to claim 6 or 7, **characterized in that**, the method comprises the following steps: preparing the derivative Dₚ of propofol represented by formula (VIII): 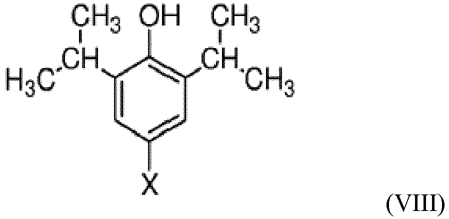
preferably, the steps comprises: dissolving propofol in an organic solvent, adding a nucleophile, a base and an oxidizing agent to prepare the propofol derivative;
more preferably, the organic solvent is methanol, the nucleophile is sodium iodide, the base is sodium hydroxide, and/or the oxidant is sodium hypochlorite;
further preferably, the reaction temperature is 0°C and/or the reaction time is 1-3 hours;
more further preferably, the reaction is quenched with sodium thiosulfate after completion.

9. The method according to claim 6, **characterized in that**, when the D_{g} is coupled with the N-terminus of gabapentin, first prepare Boc-gabapentin and then react with Dₚ;
preferably, the preparation method of the Boc-gabapentin comprises the following steps: stirring and suspending the gabapentin in an organic solvent, adding NaHCOs and BoczO in turn to react to obtain the Boc-gabapentin;
more preferably, the organic solvent is preferably 1:1 tetrahydrofuran/water; the NaHCOs is used in an amount of 1 to 5 molar equivalents, preferably 3 molar equivalents; and/or the Boc₂O is used in an amount of 1 to 2 molar equivalents, preferably 1.1 molar equivalents.

10. The method according to any one of claims 6 to 9, **characterized in that**, the method further comprises the step of extracting the crude product with an organic solvent;
preferably, the organic solvent is selected from one or more of the following: dichloromethane, chloroform, diethyl ether, methanol, ethanol, isopropanol, benzene, toluene; preferably dichloromethane.

11. A pharmaceutical composition, **characterized in that**, the pharmaceutical composition comprises the drug conjugate or a pharmaceutically acceptable salt thereof according to any one of claims 1 to 5 or the drug conjugate or a pharmaceutically acceptable salt thereof prepared by the preparation method according to any one of claims 6 to 10;
preferably, the dosage form of the pharmaceutical composition is selected from one or more of the following: tablets, capsules, injections, liposomes, and polymer microspheres.

12. Use of the drug conjugate or a pharmaceutically acceptable salt thereof according to any one of claims 1 to 5 or the drug conjugate or a pharmaceutically acceptable salt thereof prepared by the preparation method according to any one of claims 6 to 10 in the preparation of medicaments for the treatment and/or prevention of central nervous system diseases;
preferably, the central nervous system disease is selected from one or more of the following: convulsions, epilepsy, migraine, pain, depression.

13. A method for treating a central nervous system disease, **characterized in that**, the method comprises: administering the drug conjugate or a pharmaceutically acceptable compound thereof according to any one of claims 1 to 5 or a pharmaceutical composition according to claim 11 to a subject in need;
preferably, the central nervous system disease is selected from one or more of the following: convulsions, epilepsy, migraine, pain, depression.

14. A medicament for the treatment and/or prevention of central nervous system diseases, **characterized in that**, the medicament comprises the drug conjugate or a pharmaceutically acceptable salt thereof according to any one of claims 1 to 5 or the drug conjugate or a pharmaceutically acceptable salt thereof prepared by the preparation method according to any one of claims 6 to 10;
preferably, the central nervous system disease is selected from one or more of the following: convulsions, epilepsy, migraine, pain, depression.
